(19) Europäisches Patentamt
European Patent Office
Office européen des brevets

(11) **EP 4 459 263 A1**

(12) **EUROPÄISCHE PATENTANMELDUNG**

(43) Veröffentlichungstag:
06.11.2024 Patentblatt 2024/45

(21) Anmeldenummer: **24172150.5**

(22) Anmeldetag: **24.04.2024**

(51) Internationale Patentklassifikation (IPC):
**G01N 21/3563** (2014.01) **B29B 17/02** (2006.01)
**B07C 5/342** (2006.01) **G01N 21/85** (2006.01)

(52) Gemeinsame Patentklassifikation (CPC):
**G01N 21/3563; B07C 5/342; B07C 5/3425;**
**B29B 17/02; G01N 33/442;** B29B 2017/0203;
B29B 2017/0279; G01N 21/359; G01N 21/9081;
G01N 2021/3155; G01N 2021/845;
G01N 2021/8592; G01N 2201/1296

(84) Benannte Vertragsstaaten:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Benannte Erstreckungsstaaten:
**BA**
Benannte Validierungsstaaten:
**GE KH MA MD TN**

(30) Priorität: **05.05.2023 DE 102023111835**

(71) Anmelder: **Sensor-Instruments Entwicklungs-**
**und**
**Vertriebs-GmbH**
**94169 Thurmansbang (DE)**

(72) Erfinder:
• **BRAUMANDL, Walter**
**94169 Thurmansbang (DE)**
• **BRAUMANDL, Benedikt**
**94169 Thurmansbang (DE)**

(74) Vertreter: **Kramer Barske Schmidtchen**
**Patentanwälte PartG mbB**
**European Patent Attorneys**
**Landsberger Strasse 300**
**80687 München (DE)**

(54) **VERFAHREN UND VORRICHTUNG ZUR DETEKTION EINES TYPS EINES KUNSTSTOFFMATERIALS**

(57) Es werden verschiedene Vorrichtungen und Verfahren zur Identifizierung unterschiedlicher, insbesondere geschwärzter Kunststoffmaterialien bereitgestellt. Gemäß einem Aspekt wird dabei ein Typ bzw. eine Art eines Kunststoffmaterials (200) dadurch bestimmt, dass Intensitäten von reflektiertem Licht im mittleren Infrarotbereich bei Wellenlängen über 2,2 $\mu$m detektiert werden. Eine Verwendung eines Heizstrahlers (20), der insbesondere in diesem Wellenlängenbereich emittiert, ermöglicht auf einfache Weise eine Anregung in dem gesamten MIR-Bereich sowie eine Detektion der Reflexion bei mehreren, beispielsweise fünf oder acht unterschiedlichen Wellenlängen bzw. Wellenlängenbereichen. Auf diese Weise können insbesondere Carbon-Black-Materialien zuverlässig voneinander unterschieden werden.

FIG 1

**Beschreibung**

Die vorliegende Erfindung bezieht sich auf Verfahren und Vorrichtungen zur Detektion eines Typs eines Kunststoffmaterials, insbesondere im Rahmen eines Recyclingprozesses.

Beim Recyceln bzw. der Wiederverwertung von Kunststoffen besteht eine der Aufgaben darin, die unterschiedlichen Kunststoffe sortenrein zu trennen. Dabei ist zu beachten, dass je nach Art der Wiederverwendung des Endprodukts nach Kunststoffart, Farbe und gegebenenfalls sogar nach dem Einsatzgebiet des Materials unterschieden werden muss. Beispielsweise sollte verhindert werden, dass das Material einer Kunststoffflasche, deren Inhalt aus Reinigungschemikalien bestand, zukünftig in der Matrix einer Kunststoffflasche für den Lebensmittelbereich wiederverwendet wird.

Es ist bekannt, zerkleinertes Kunststoffmaterial über ein Förderband zuzuführen und mittels eines Linienscanners in Form einer Infrarotkamera Spektren aufzuzeichnen, die anschließend ausgewertet werden. Der in einem Segment mittels spezifischem Infrarotspektrum erkannte Kunststoff wird dann im Anschluss entsprechend aussortiert.

Die oben genannten Infrarotkameras sind jedoch zum einen in der Anschaffung relativ kostenintensiv, und zum anderen können sie nicht an jedem Ort eingesetzt werden. Beispielsweise ist an Rücknahmestationen in Supermärkten, an denen das Recyclinggut als ganze Kunststoffobjekte einzeln zugeführt wird, eine Separation solcher einzelnen Objekte erforderlich.

Aus der DE 10 2020 113 252 A1 sind ein Verfahren und eine Vorrichtung zur Sortierung von Kunststoffobjekten bekannt, bei denen ein Typ von Kunststoffobjekten durch Detektion der Intensitäten von reflektiertem Licht im Infraroten bei unterschiedlichen Wellenlängen bestimmt wird. Dabei werden IR-Wellenlängen im Bereich zwischen 1000 nm und 2200 nm, insbesondere 1000 nm bis 1700 nm verwendet.

WO 2023/ 017 639 A1 offenbart ein Sortiersystem für Kunststoffabfälle und ein zugehöriges Verfahren.

WO 2022/ 170 262 A1 offenbart ein weiteres Verfahren zum Sortieren von Kunststoffen.

DE 693 07 344 T2 betrifft ebenfalls die Trennung von Kunststoffen.

Es ist eine Aufgabe der Erfindung, ein Verfahren und eine Vorrichtung zur Detektion eines Typs eines Kunststoffmaterials bereitzustellen, die auf möglichst zuverlässige und einfache Weise einen Typ, insbesondere eine Art, eines Kunststoffmaterials detektieren können. Diese Aufgabe wird gelöst durch ein Verfahren nach Anspruch 1 und eine Vorrichtung nach Anspruch 7. Weiterbildungen der Erfindung sind in den abhängigen Ansprüchen angegeben.

Weitere Aspekte der vorliegenden Offenbarung sind:

1. Verfahren zur Detektion eines Typs eines Kunststoffmaterials (200), insbesondere im Rahmen eines Recyclingprozesses, mit

Aussenden von Anregungslicht, das IR-Licht in einem MIR-Bereich zwischen etwa 2,2 $\mu$m und etwa 5 $\mu$m aufweist, in Richtung des Kunststoffmaterials,
Detektieren von MIR-Intensitäten IR1, ..., IRn von an dem Kunststoffmaterial reflektiertem IR-Licht in mindestens zwei unterschiedlichen MIR-Wellenlängenbereichen, die in dem MIR-Bereich zwischen etwa 2,2 $\mu$m und etwa 5 $\mu$m liegen,
Bestimmen des Typs des Kunststoffmaterials durch Vergleichen mindestens der detektierten MIR-Intensitäten mit einer Mehrzahl von Referenzgrößen, die jeweils unterschiedlichen Typen von Kunststoffmaterial zugeordnet sind, und Bestimmen der Referenzgröße mit der größten Übereinstimmung als den Typ des Kunststoffmaterials angebend.

2. Verfahren nach Aspekt 1, bei dem anhand der detektierten MIR-Intensitäten normierte Intensitäten ir1, ..., irn und optional ein Normierungsfaktor B bestimmt werden, die gegeben sind durch:

$$ir1 = IR1/(IR1 + \ldots + IRn)$$

$$\ldots$$

$$irn = IRn/(IR1 + \ldots + IRn)$$

$$B = (IR1 + \ldots + IRn)/n$$

und bei dem der Typ des Kunststoffmaterials durch Vergleichen einer Kombination aus den normierten IR-Intensitäten und optional dem Normierungsfaktor mit der Mehrzahl von Referenzgrößen bestimmt wird.

3. Verfahren nach Aspekt 1 oder 2, ferner mit
Aussenden des IR-Lichts unter Verwendung einer breitbandigen IR-Lichtquelle (20) und Detektieren der MIR-Intensitäten durch unterschiedliche, jeweils den mindestens zwei MIR-Wellenlängenbereichen zugeordnete IR-Detektionsvorrichtungen (16-19, 21), beispielsweise IR-Fotodioden.

4. Verfahren nach einem der Aspekte 1 bis 3, bei dem mindestens drei, insbesondere vier, unterschiedliche MIR-Wellenlängenbereiche verwendet werden, die jeweils unterschiedliche Zentralwellenlängen aufweisen, die ausgewählt sind aus 3,3 $\mu$m, 3,9 $\mu$m, 4,26 $\mu$m und 4,45 $\mu$m.

5. Verfahren nach einem der Aspekte 1 bis 4, bei dem einer der mindestens zwei unterschiedlichen MIR-Wellenlängenbereiche im Wesentlichen der gesamte MIR-Bereich zwischen etwa 2,2 $\mu$m und etwa 5 $\mu$m ist.

6. Verfahren nach einem der Aspekte 1 bis 5, bei dem das Anregungslicht ferner Licht im NIR-Bereich und/oder sichtbaren Bereich aufweist, ferner mit

Detektieren von einer oder mehreren, beispielsweise drei, weiteren Intensitäten I1, ..., In von reflektiertem Licht in dem NIR-Bereich und/oder sichtbaren Bereich und
Bestimmen des Typs des Kunststoffmaterials durch Vergleichen der detektierten MIR-Intensitäten und der detektierten weiteren Intensitäten mit der Mehrzahl von Referenzgrößen.

7. Verfahren nach einem der Aspekte 1 bis 6, ferner mit

Bestimmen einer Strahlungsleistung des Anregungslichts, beispielsweise basierend auf einer durch einen Temperatursensor (30) erfassten Temperatur, und/oder
Detektieren eines Spektrums des Anregungslichts, ferner mit
Steuern des ausgesandten Anregungslichts basierend auf der bestimmten Strahlungsleistung und/oder dem detektierten Spektrum zum Erhalten eines Soll-Anregungslichts oder
Modifizieren der detektierten MIR-Intensitäten basierend auf der bestimmten Strahlungsleistung und/oder dem detektierten Spektrum vor dem Vergleichen mit der Mehrzahl von Referenzgrößen.

8. Verfahren nach einem der Aspekte 1 bis 7, ferner mit
Sortieren des Kunststoffmaterials (200) basierend auf dem bestimmten Typ.

9. Vorrichtung (100) zur Detektion eines Typs eines Kunststoffmaterials (200), insbesondere im Rahmen eines Recyclingprozesses, mit
einer Sendeeinheit (10) zum Aussenden von Anregungslicht, das IR-Licht in einem MIR-Bereich zwischen etwa 2,2 $\mu$m und etwa 5 $\mu$m aufweist, in Richtung des Kunststoffmaterials, einer IR-Detektionseinheit (12) zum Detektieren von MIR-Intensitäten IR1, ..., IRn von an dem Kunststoffmaterial reflektiertem IR-Licht in mindestens zwei unterschiedlichen MIR-Wellenlängenbereichen, die in dem MIR-Bereich zwischen etwa 2,2 $\mu$m und etwa 5 $\mu$m liegen, einer Steuereinheit (14) zum Bestimmen des Typs des Kunststoffmaterials durch Vergleichen mindestens der detektierten MIR-Intensitäten mit einer Mehrzahl von Referenzgrößen, die jeweils unterschiedlichen Typen von Kunststoffmaterial zugeordnet sind, und Bestimmen der Referenzgröße mit der größten Übereinstimmung als den Typ des Kunststoffmaterials angebend.

10. Vorrichtung nach Aspekt 9, bei der die Sendeeinheit (10) eine breitbandige IR-Lichtquelle (20) aufweist und die IR-Detektionseinheit (12) unterschiedliche, jeweils den mindestens zwei MIR-Wellenlängenbereichen zugeordnete IR-Detektionsvorrichtungen (16, 18), beispielsweise IR-Fotodioden, aufweist.

11. Vorrichtung nach Aspekt 10, bei der die breitbandige IR-Lichtquelle (20) ein Infrarot-Heizstrahler ist, der sich in einer Längsrichtung (y) erstreckt, insbesondere ein Quarzstrahler, der einen in ein mit Inertgas gefülltes Quarzglasrohr integrierten Heizwiderstand (22) aufweist, oder ein flächiger Infrarotstrahler, beispielsweise ein KRELUS-Infrarotstrahler.

12. Vorrichtung nach Aspekt 10 oder 11, bei der die Sendeeinheit (10) einen Reflektor (24) aufweist, der von der IR-Lichtquelle (20) emittiertes Licht in Richtung eines Detektionsbereichs (26) der Vorrichtung (100) lenkt, beispielsweise einen Parabolspiegel, der die IR-Lichtquelle (20) teilweise umgibt, und/oder mehrere Bleche, die in Richtung des Detektionsbereichs aufeinander zulaufen, so dass ein schlitzförmiger Austrittsbereich (28) der Sendeeinheit (10) erhalten wird.

13. Vorrichtung nach einem der Aspekte 10 bis 12, ferner mit mindestens einem Temperatursensor (30),

bei der die Steuereinheit (14) angepasst ist zum Bestimmen einer Strahlungsleistung des Anregungslichts basierend auf einer durch den mindestens einen Temperatursensor (30) erfassten Temperatur und Steuern der IR-Lichtquelle (20) basierend auf der bestimmten Strahlungsleistung zum Erhalten eines Soll-Anregungslichts oder Modifizieren der detektierten MIR-Intensitäten basierend auf der bestimmten Strahlungsleistung vor dem Vergleichen mit der Mehrzahl von Referenzgrößen.

14. Vorrichtung nach einem der Aspekte 10 bis 13, ferner mit mindestens einem Detektor (32), der dazu angepasst ist, ein Spektrum des Anregungslichts zu detektieren,
bei der die Steuereinheit (14) angepasst ist zum Steuern des ausgesandten Anregungslichts basierend auf dem detektierten Spektrum zum Erhalten eines Soll-Anregungslichts oder Modifizieren der detektierten MIR-Intensitäten basierend auf dem detektierten Spektrum vor dem Vergleichen mit der Mehrzahl von Referenzgrößen.

15. Vorrichtung nach einem der Aspekte 10 bis 14, bei der die Detektionseinheit (12) mindestens drei, insbesondere vier, IR-Detektionsvorrichtungen (16, 17, 18, 19) aufweist, die jeweils eine Detektion in den unterschiedlichen MIR-Wellenlängenbereichen durchführen, beispielsweise bei 3,3 $\mu$m, 3,9 $\mu$m, 4,26 $\mu$m und/oder 4,45 $\mu$m.

16. Vorrichtung nach einem der Aspekte 10 bis 15, bei der die Detektionseinheit (12) eine IR-Detektionsvorrichtung (21) aufweist, die eine Detektion über im Wesentlichen den gesamten MIR-Bereich zwischen etwa 2,2 $\mu$m und etwa 5 $\mu$m durchführt.

17. Vorrichtung nach einem der Aspekte 10 bis 16, bei der die Detektionseinheit (12) eine oder mehrere IR-Detektionsvorrichtungen (23, 25), die eine Detektion im NIR-Bereich, beispielsweise zwischen 0,9 $\mu$m und 1,7 $\mu$m oder zwischen 1,7 $\mu$m und 2,55 $\mu$m, durchführen, und/oder eine Detektionsvorrichtung (27) für sichtbares Licht aufweist, wobei der Vergleich mit den Referenzwerten zusätzlich auf den durch diese weiteren Detektionsvorrichtungen detektierten Intensitäten basiert.

18. Vorrichtung nach einem der Aspekte 10 bis 17, bei der die IR-Detektionseinheiten (16-19, 21), beispielsweise Fotodioden, in Form einer Matrix auf einer gemeinsamen Platine (34) angeordnet sind.

19. Vorrichtung nach einem der Aspekte 9 bis 18, bei der die Sendeeinheit (10) und die IR-Detektionseinheit (12) in einem gemeinsamen Gehäuse (40) integriert sind, insbesondere mit einem an dem Gehäuse (40) ausgebildeten Befestigungsbereich (41), der zur Befestigung an einem Behälter (42) für das Kunststoffmaterial (200) ausgebildet ist, und einem innerhalb des Befestigungsbereichs (41) vorgesehenen Lichtaustritts- bzw. Lichteintrittsbereich.

20. Vorrichtung nach einem der Aspekte 9 bis 19, ferner mit einer Optik (50), die vor der IR-Detektionseinheit (12) vorgesehen ist, insbesondere mit einer oder mehreren Linsen (52), die beispielsweise Si, $BaF_2$ oder $CaF_2$ aufweisen oder daraus bestehen.

21. Vorrichtung nach Aspekt 20, bei der drei Linsen (52a, 52b, 52c) vorgesehen sind, von denen zwei äußere in Bezug auf die innere geneigt sind, ferner mit zwei Umlenkspiegeln (56), die jeweils den beiden äußeren Linsen zugeordnet sind, zum Lenken des reflektierten IR-Lichts auf einen bestimmten, beispielsweise äußeren, Bereich der IR-Detektionseinheit (12).

22. Vorrichtung nach einem der Aspekte 9 bis 21, ferner mit einer Sortiereinheit (60), die dazu angepasst ist, von der Steuereinheit (14) basierend auf dem Ergebnis der Bestimmung des Typs des Kunststoffmaterials (200) zum Sortieren desselben angesteuert zu werden.

23. Vorrichtung nach einem der Aspekte 9 bis 22, bei der mehrere IR-Detektionseinheiten (12) in einer Richtung benachbart zueinander angeordnet sind, so dass eine räumlich aufgelöste Detektion erhalten wird.

Während es mit den aus der DE 10 2020 113 252 A1 bekannten Verfahren und Vorrichtungen möglich ist, viele verschiedene Arten von Kunststoffobjekten beispielsweise nach ihrer Farbe, ihrem Verwendungszweck und ihrem Basismaterial zu sortieren, gibt es dennoch einige Kunststoffarten, bei denen diese bekannten Verfahren bzw. Vorrichtungen Probleme aufweisen. So weisen insbesondere rußhaltige Kunststoffe, d.h., im Wesentlichen schwarze oder sehr dunkel eingefärbte Kunststoffe (sogenanntes Carbon-Black-Material), die Eigenschaft auf, dass auch Licht im Nahinfrarotbereich (NIR-Bereich) in erheblichem Maße absorbiert wird. Das heißt, die reflektierten Intensitäten reichen nicht aus, um mit den bekannten Verfahren und Vorrichtungen zuverlässig unterschiedliche Materialien mit solchen Eigenschaften zu identifizieren bzw. zu unterscheiden.

Der vorliegenden Erfindung liegt zumindest zum Teil die Erkenntnis zugrunde, dass eine zuverlässige Unterscheidung von Carbon-Black-Materialien, die unterschiedliche Kunststoffbasismaterialien aufweisen, dadurch ermöglicht werden kann, dass Wellenlängen im mittleren Infrarotbereich (MIR-Bereich) verwendet werden, um von den Kunststoffen reflektiertes Licht zu detektieren. Dabei weisen unterschiedliche Kunststoffmaterialien wie beispielsweise PET, POM, PA6, PE300, PVC und Acryl bei unterschiedlichen MIR-Wellenlängen unterschiedliche Absorptions- bzw. Reflexionseigenschaften auf, anhand derer eine Unterscheidung zwischen den Materialien möglich ist. Auf ähnliche Weise kann beispielsweise auch zwischen Kunststoffmaterialien mit ähnlichen, relativ dunklen Farben unterschieden werden, beispielsweise blaues PET von grauem PET oder schwarzem PET.

Weiter liegt der vorliegenden Erfindung die Erkenntnis zugrunde, dass auf besonders einfache Weise eine Anregung bei verschiedenen MIR-Wellenlängen erhalten werden kann, indem ein herkömmlicher Infrarot-Heizstrahler als Anregungslichtquelle verwendet wird. Dabei hat sich als besonders vorteilhaft erwiesen, eine Lichtführungseinrichtung zum Leiten des emittierten Anregungslichts hin zu dem Messobjekt einzusetzen. Besonders vorteilhaft ist dabei, die Sensorik, die das reflektierte Licht erfasst, durch diese Lichtführungseinrichtung von der Anregungslichtquelle abzuschirmen, so dass kein direkter Lichteinfall auf die Sensorik erfolgen kann.

Es wurde ferner festgestellt, dass bei einer Verwendung einer geeigneten MIR-Lichtquelle auch bei im sichtbaren Bereich als beispielsweise schwarz wahrgenommenen Objekten reflektierte Signale erhalten werden können, die in Bezug auf die Signalstärke vergleichbar sind mit im sichtbaren Bereich als hell interpretierten Objekten. Dies ermöglicht eine zuverlässige Unterscheidung der unterschiedlichen Kunststoffmaterialien, da die Signalpegel eine ausreichende Stärke aufweisen.

Für eine möglichst zuverlässige Detektion hat es sich als vorteilhaft erwiesen, mehrere unterschiedliche Wellenlängenbereiche zu verwenden, beispielsweise fünf oder sogar acht unterschiedliche Wellenlängenbereiche, von denen jedoch nicht alle im MIR-Bereich liegen müssen. In jedem Fall sollten jedoch mindestens zwei, bevorzugt drei bis fünf der detektierten Intensitäten im MIR-Bereich liegen, um insbesondere dunkle bzw. schwarze Kunststoffe unterschiedlicher Art zuverlässig voneinander unterscheiden zu können.

Die Unterscheidung der einzelnen Kunststoffmaterialien erfolgt bevorzugt durch Vergleich eines aus den detektieren Intensitäten gebildeten Vektors mit mehreren Referenzvektoren auf an sich bekannte Weise. Der Referenzvektor, der den geringsten Abstand zu dem Vektor der Detektionswerte aufweist, wird als den Typ bzw. die Art des Kunststoffmaterials angebend betrachtet. Dabei ist ebenfalls vorgesehen, beispielsweise im Rahmen einer Lernprozedur oder während des normalen Betriebs die Referenzvektoren zu optimieren, möglicherweise unter Verwendung einer KI und/oder eines neuronalen Netzes.

Ferner wurde erkannt, dass zur Signaloptimierung vorteilhafter Weise eine Optik vor der Detektionseinheit angeordnet werden sollte, die beispielsweise das reflektierte Licht auf bestimmte Bereiche einer Detektormatrix lenken kann, um so die Lichtausbeute zu optimieren bzw. möglichst große Detektionssignale zu erhalten. Aufgrund des verwendeten Wellenlängenbereichs sind dabei spezielle Gläser, beispielsweise $CaF_2$ oder $BaF_2$ enthaltend, oder Linsen etwa aus Si erforderlich, um die Transmission des reflektierten Lichts zu gewährleisten.

Insbesondere bei einer Detektion an Kunststoffgranulat, das üblicherweise in Behältern transportiert wird, hat es sich als vorteilhaft erwiesen, die hierin beschriebenen Sende- bzw. Detektionseinheiten in ein gemeinsames Gehäuse zu integrieren, das dann an dem entsprechenden Behälter angebracht (beispielsweise angeflanscht) werden kann. Diesbezüglich ist bevorzugt in dem Behälter ein Fenster mit einem für MIR-Strahlung transparenten Glas vorgesehen. Auf diese Weise kann insbesondere auch ein vorgegebener Abstand zwischen Kunststoffmaterial und Sende- und Empfangseinheit gewährleistet werden. Ferner kann die Sensorik auch beispielsweise an Dosiereinheiten zur Rezyklatkontrolle unmittelbar vor der Zufuhr des Rezyklats zu Weiterverarbeitungsanlagen wie Spritzgussanlagen und dergleichen angebracht werden.

Es ist ferner vorgesehen, die hierin beschriebenen Verfahren und Vorrichtungen zur Sortierung von unterschiedlichen Kunststoffen zu verwenden. Dabei können beispielsweise über einem Förderband oder einer ähnlichen Vorrichtung mehrere der hierin beschriebenen Detektionseinheiten in transversaler Richtung nebeneinander angeordnet werden, um eine räumliche Auflösung einer Detektion eines Typs von unterschiedlichen Kunststoffobjekten zu ermöglichen. Ansprechend auf die Detektion kann dann eine stromabwärts angeordnete Sortiervorrichtung auf an sich bekannte Weise angesteuert werden, um die unterschiedlichen Kunststoffobjekte beispielsweise in unterschiedliche Behälter zu befördern.

Weitere Merkmale und Zweckmäßigkeiten ergeben sich aus der Beschreibung von Ausführungsbeispielen anhand der Figuren. Es zeigen:

Fig. 1 eine schematische Vorderansicht einer Vorrichtung gemäß der vorliegenden Erfindung;

Fig. 2 eine schematische Seitenansicht der in Fig. 1 gezeigten Vorrichtung;

Fig. 3 eine schematische Draufsicht auf eine Detektionseinheit gemäß der vorliegenden Erfindung;

Fig. 4a und 4b graphische Darstellungen, die Wellenlängenbereiche zeigen, die für eine Detektion verwendet werden;

Fig. 5 einen Ausschnitt, der eine in einem Gehäuse aufgenommene Vorrichtung gemäß der vorliegenden Erfindung zeigt, die an einem Behälter für Kunststoffmaterial angebracht ist;

Fig. 6 eine schematische Vorderansicht einer weiteren Ausführungsform einer Vorrichtung gemäß der vorliegenden Erfindung;

Fig. 7 eine Draufsicht auf eine weitere Detektionseinheit gemäß der vorliegenden Erfindung; und

Fig. 8 eine schematische perspektivische Ansicht einer Vorrichtung gemäß der vorliegenden Erfindung, die zur Sortierung von Kunststoffobjekten verwendet wird.

Im Folgenden werden Ausführungsformen einer Vorrichtung zur Detektion eines Typs eines Kunststoffmaterials sowie zugehörige Verfahren unter Bezugnahme auf die Figuren beschrieben.

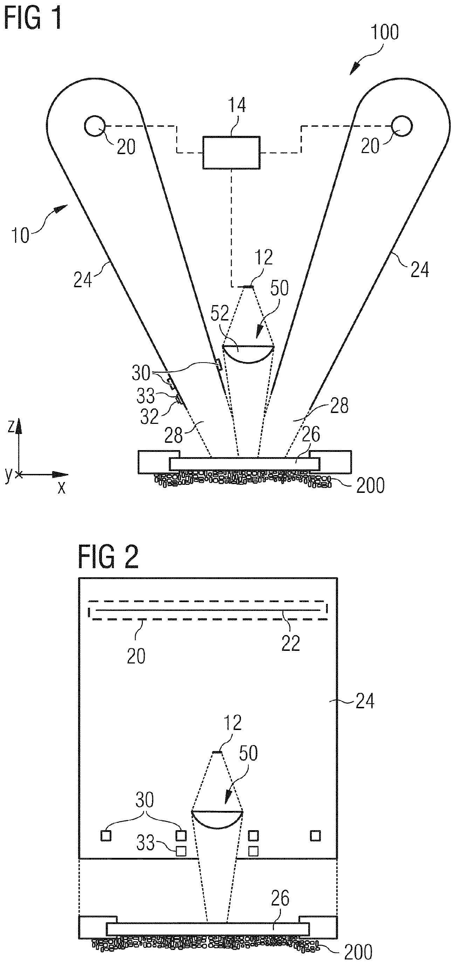

Fig. 1 zeigt eine schematische Vorderansicht einer ersten Ausführungsform einer Vorrichtung 100 zur Detektion eines Typs eines Kunststoffmaterials 200, insbesondere im Rahmen eines Recyclingprozesses, gemäß der vorliegenden Erfindung. Die Vorrichtung 100 weist eine Sendeeinheit 10 zum Aussenden von Anregungslicht, das IR-Licht in einem MIR-Bereich zwischen etwa 2,2 $\mu$m und etwa 5 $\mu$m aufweist, in Richtung des Kunststoffmaterials sowie eine IR-Detektionseinheit 12 zum Detektieren von MIR-Intensitäten IR1, ..., IRn von an dem Kunststoffmaterial reflektiertem IR-Licht in mindestens zwei unterschiedlichen MIR-Wellenlängenbereichen, die in dem MIR-Bereich zwischen etwa 2,2 $\mu$m und etwa 5 $\mu$m liegen, auf. Dabei versteht sich, dass der Bereich zwischen etwa 2,2 $\mu$m und etwa 5 $\mu$m lediglich beispielhaft ist, und bei Bedarf auch andere Bereiche, insbesondere Teilbereiche des angegebenen Bereichs, verwendet werden können. Beispielsweise wäre auch ein Bereich zwischen etwa 3,3 $\mu$m und etwa 4,5 $\mu$m vorstellbar.

Die Vorrichtung 100 weist ferner eine Steuereinheit 14 zum Bestimmen des Typs des Kunststoffmaterials durch Vergleichen mindestens der detektierten MIR-Intensitäten mit einer Mehrzahl von Referenzgrößen, die jeweils unterschiedlichen Typen von Kunststoffmaterial zugeordnet sind, und Bestimmen der Referenzgröße mit der größten Übereinstimmung als den Typ des Kunststoffmaterials angebend auf.

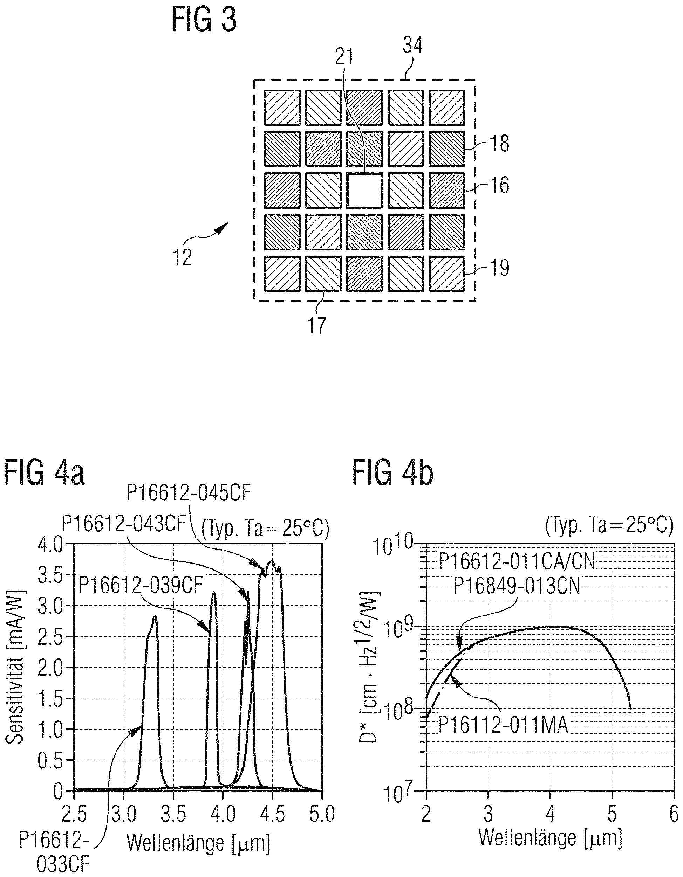

Bei der in Fig. 1 gezeigten Ausführungsform weist die Sendeeinheit 10 eine breitbandige IR-Lichtquelle 20 auf. Ferner weist die IR-Detektionseinheit 12 unterschiedliche, jeweils den mindestens zwei MIR-Wellenlängenbereichen zugeordnete IR-Detektionsvorrichtungen 16, 18, beispielsweise IR-Fotodioden, auf (siehe Fig. 3). Insbesondere ist die breitbandige IR-Lichtquelle 20 beispielsweise ein bekannter Infrarot-Heizstrahler, der sich in einer Längsrichtung y erstreckt. Beispielsweise kann ein Quarzstrahler verwendet werden, der einen in ein mit Inertgas gefülltes Quarzglasrohr integrierten Heizwiderstand 22 aufweist, wie dies in der Seitenansicht in Fig. 2 schematisch dargestellt ist. Alternativ kann ein flächiger, ggf. modular aufgebauter Infrarotstrahler, beispielsweise ein bekannter KRELUS-Infrarotstrahler, verwendet werden, der z.B. mehrere Metallfolien aufweist.

Wie in Fig. 1 gezeigt, weist bei der vorliegenden Ausführungsform die Sendeeinheit 10 eine Lichtleiteinheit wie einen Reflektor 24 auf, der von der IR-Lichtquelle 20 emittiertes Licht in Richtung eines Detektionsbereichs 26 der Vorrichtung 100 lenkt. Beispielsweise kann der Reflektor 24 einen Parabolspiegel, der die IR-Lichtquelle 20 teilweise umgibt, und/oder mehrere Bleche, die in Richtung des Detektionsbereichs 26 aufeinander zu laufen, so dass ein schlitzförmiger Austrittsbereich 28 der Sendeeinheit 10 erhalten wird, aufweisen. Dieser schlitzförmige Austrittsbereich 28 verläuft dabei, wie in Fig. 1 und 2 gezeigt, im Wesentlichen in der Längsrichtung y des Strahlers.

Bei dem in Fig. 1 gezeigten Beispiel sind zwei identisch ausgebildete Sendeeinheiten 10 in einer zur Längsrichtung y senkrechten transversalen Richtung x bezüglich des Detektionsbereichs 26 auf gegenüberliegenden Seiten vorgesehen. Es ist ersichtlich, dass dadurch die Lichtausbeute erhöht werden kann. Es versteht sich jedoch, dass bei anderen Ausführungsformen lediglich eine der Sendeeinheiten 10 vorgesehen sein kann.

Wie in Fig. 1 gezeigt, ist die IR-Detektionseinheit 12 so angeordnet, dass Anregungslicht nicht direkt auf diese treffen kann. Dies kann beispielsweise wie in Fig. 1 gezeigt dadurch erreicht werden, dass die IR-Detektionseinheit 12 bezüglich der IR-Lichtquelle 20 auf der äußeren Seite des Reflektors 24 angeordnet ist, beispielsweise zwischen den Reflektoren 24 der gegenüberliegend zueinander angeordneten Sendeeinheiten 10.

Wie in Fig. 1 schematisch angedeutet, ist die Steuereinheit 14 durch geeignete Steuerleitungen mit sowohl der Sendeeinheit 10 (der IR-Lichtquelle 20) als auch der IR-Detektionseinheit 12 verbunden. Die Steuereinheit 14 steuert die Sendeeinheit 10 bzw. IR-Lichtquelle 20 sowie die IR-Detektionseinheit 12 auf bekannte Weise an bzw. erhält Signale von diesen.

Wie in Fig. 1 und 2 gezeigt, weist die Vorrichtung 100 ferner mindestens einen Temperatursensor 30 auf. Dabei ist die Steuereinheit 14 angepasst zum Bestimmen einer Strahlungsleistung des Anregungslichts basierend auf einer durch den mindestens einen Temperatursensor 30 erfassten Temperatur und Steuern der IR-Lichtquelle 20 basierend auf der bestimmten Strahlungsleistung zum Erhalten eines Soll-Anregungslichts oder Modifizieren der detektierten MIR-Intensitäten basierend auf der bestimmten Strahlungsleistung vor dem Vergleichen mit den im Voraus hinterlegten Referenzgrößen. Der Grund für das Vorsehen des Temperatursensors 30 ist der, dass insbesondere bei Verwendung eines breitbandigen Strahlers das Emissionsspektrum desselben von verschiedenen Betriebsparametern bzw. insbesondere der Temperatur desselben abhängt. Damit jedoch ein Vergleich mit den im Voraus hinterlegten Referenzwerten sinnvoll ist, sollte das Emissionsspektrum bekannt sein bzw. das Anregungslicht ein Soll-Anregungslicht sein. Daher können im Voraus unterschiedliche, von dem Temperatursensor bzw. den Temperatursensoren 30 gemessene Temperaturen unterschiedlichen Emissionsspektren der IR-Lichtquelle 20 zugeordnet werden. Das heißt, es kann eine Korrelation zwischen der von dem Temperatursensor 30 gemessenen Temperatur und der Temperatur des Strahlers 20 verwendet werden, um das Emissionsspektrum zu bestimmen. Die Steuereinheit 14 kann dann dazu angepasst sein, entweder den Strahler 20 geeignet anzusteuern, so dass der Temperatursensor 30 eine vorbestimmte Temperatur misst, die dem Soll-Anregungslicht entspricht, oder alternativ die durch die Detektionseinheit 12 detektierten MIR-Intensitäten so zu modifizieren bzw. neu zu berechnen, dass sie den Referenzwerten entsprechen, die für ein bestimmtes Emissionsspektrum erhalten wurden. Dem Fachmann ist ohne weiteres geläufig, wie entsprechende Berechnungen bzw. Ansteuerungen durchgeführt werden müssen, so dass auf eine weitere Erläuterung verzichtet wird.

Alternativ oder zusätzlich zu den ein oder mehreren Temperatursensoren 30 können ebenfalls ein oder mehrere Detektoren 32 vorgesehen sein, beispielsweise ebenfalls an dem Reflektor 24, die dazu angepasst sind, ein Spektrum des Anregungslichts zu detektieren. Auf ähnliche Weise wie auf der Basis der Detektion durch den Temperatursensor 30 kann die Steuereinheit 14 dann auf der Basis des detektierten Spektrums das ausgesandte Anregungslicht zum Erhalten des Soll-Anregungslichts steuern oder die detektierten MIR-Intensitäten vor dem Vergleichen mit den mehreren Referenzgrößen modifizieren.

Es versteht sich, dass bei Ausführungsformen, bei denen anstelle der breitbandigen IR-Lichtquelle 20 beispielsweise einzelne Emissionsvorrichtungen, die einen bestimmten Emissionsbereich, gegebenenfalls mit konstanter Intensität der Emission, aufweisen, verwendet werden, unter Umständen auf den Temperatursensor 30 und/oder den Detektor 32 verzichtet werden kann.

Bei der vorliegenden Ausführungsform sind jedoch sowohl der Temperatursensor 30 als auch der Detektor 32 an dem Reflektor 24 vorgesehen. Es versteht sich, dass für den Detektor 32 eine geeignete Öffnung, die beispielsweise durch ein geeignetes Abdeckglas 33 bedeckt ist, in dem Reflektor 24 vorhanden sein muss. Der Detektor 32 kann beispielsweise dazu angepasst sein, eine Detektion für bestimmte Wellenlängen durchzuführen, die auch der späteren Detektion durch die IR-Detektionseinheit 12 zugrunde liegen.

Fig. 3 zeigt ein Beispiel für die IR-Detektionseinheit 12 gemäß der vorliegenden Erfindung. Allgemein weist die IR-Detektionseinheit 12 mindestens drei, insbesondere vier, IR-Detektionsvorrichtungen 16, 17, 18, 19 auf, die jeweils eine Detektion in den unterschiedlichen MIR-Wellenlängenbereichen durchführen. Beispielsweise können Wellenlängen von 3,3 $\mu$m, 3,9 $\mu$m, 4,26 $\mu$m und/oder 4,45 $\mu$m verwendet werden. Es versteht sich, dass dies beispielsweise durch Vorsehen geeigneter Bandpassfilter vor jeweiligen Fotodioden erzielt werden kann. Fig. 4a zeigt beispielhaft die Sensitivität der vier Detektionsvorrichtungen 16-19 bei den genannten Wellenlängen.

Wie in Fig. 3 gezeigt, sind erfindungsgemäß die IR-Detektionsvorrichtungen in Form einer Matrix auf einer gemeinsamen Platine 34 angeordnet. Dabei sind für jede Detektionsvorrichtung die entsprechenden Fotodioden mehrmals in der Matrix vorhanden. Auf diese Weise kann durch Aufsummieren der detektierten Intensitäten eine erhöhte Signalstärke erzielt werden, die den Vergleich bzw. die Auswertung erleichtert.

Bei der in Fig. 3 gezeigten Ausführungsform weist die Detektionseinheit 12 ferner eine IR-Detektionsvorrichtung 21 auf, die eine Detektion über im Wesentlichen den gesamten MIR-Bereich zwischen etwa 2,2 $\mu$m und etwa 5 $\mu$m durchführt. Durch eine Integration der detektierten Spektralantwort in diesem Bereich kann eine weitere Größe erhalten werden, die in den Vergleich mit den Referenzvektoren eingeht. Aufgrund des breiten Detektionsbereichs kann es ausreichend sein, lediglich eine der IR-Detektionsvorrichtungen 21 in der in Fig. 3 gezeigten Matrix vorzusehen. Fig. 4b zeigt die Spektralantwort der IR-Detektionsvorrichtung 21 bei unterschiedlichen Wellenlängen. Es versteht sich, dass für den Vergleich eine geeignete Normierung und/oder Mittelung der detektierten Spektralantwort bzw. Intensität verwendet werden kann.

Unter Verwendung der in Fig. 1 und 2 gezeigten Vorrichtung, insbesondere der in Fig. 3 gezeigten IR-Detektionseinheit 12, kann nun eine Detektion bzw. Bestimmung eines Typs, insbesondere eines Materials, unterschiedlicher Kunststoffe durch Vergleich mit geeigneten Referenzvektoren durchgeführt werden. Dies wird auch als sogenanntes Mehrbereichsverfahren bezeichnet. Das heißt, mittels der in Fig. 3 gezeigten Detektionseinheit 12 werden vier Intensitätswerte bei den jeweiligen Wellenlängen im MIR-Bereich und ein integrierter (ggf. normierter oder gemittelter) Wert über den gesamten Bereich erhalten. Diese MIR-Intensitäten können dann mit den Referenzvektoren, die ebenfalls entsprechende Intensitäten aufweisen, in einem in diesem Fall fünfdimensionalen Vektorraum verglichen werden. Dabei wird insbesondere der euklidische Abstand zwischen dem Detektionsvektor und den unterschiedlichen Referenzvektoren bestimmt, und derjenige Referenzvektor mit dem geringsten Abstand wird als der mit dem Detektionsvektor übereinstimmende Vektor angesehen. Diesem übereinstimmenden Referenzvektor ist dann ein bestimmter Typ, beispielsweise ein bestimmtes Material, des Kunststoffs zugeordnet. Beispielsweise können den einzelnen Referenzvektoren unterschiedliche Kunststoffarten wie PET, POM, PA6, PE300 und dergleichen oder unterschiedliche Farben, beispielsweise blau, grau, schwarz, ein und desselben Kunststoffmaterials zugeordnet sein. Es versteht sich, dass die Referenzvektoren dadurch bestimmt werden können, dass für jede von bekannten Kunststoffarten die jeweiligen Intensitäten detektiert werden und dann daraus der zugehörige Referenzvektor gebildet wird. Wie eingangs bereits erwähnt, können die so einmal erzeugten Referenzvektoren beispielsweise im Rahmen einer Lernprozedur oder während des normalen Betriebs optimiert bzw. angepasst werden, möglicherweise unter Verwendung einer KI und/oder eines neuronalen Netzes, um so die Unterscheidung der einzelnen Kunststoffarten noch zuverlässiger zu gestalten.

Vorteilhaft können dabei normierte Intensitäten IR1, ... IRn und optional ein Normierungsfaktor B verwendet werden. Die normierten Intensitäten bzw. der Normierungsfaktor B sind gegeben durch folgende Ausdrücke:

$$\mathrm{ir}1 = \mathrm{IR}1/(\mathrm{IR}1 + \dots + \mathrm{IR}n)$$

$$\dots$$

$$\mathrm{ir}n = \mathrm{IR}n/(\mathrm{IR}1 + \dots + \mathrm{IR}n)$$

$$B = (\mathrm{IR}1 + \dots + \mathrm{IR}n)/n \ (\mathrm{oder}\ B = \mathrm{IR}1 + \dots + \mathrm{IR}n)$$

Auf diese Weise kann der Typ des Kunststoffmaterials durch Vergleichen einer Kombination aus den normierten IR-Intensitäten und optional dem Normierungsfaktor mit der Mehrzahl von Referenzgrößen bzw. Referenzvektoren bestimmt werden.

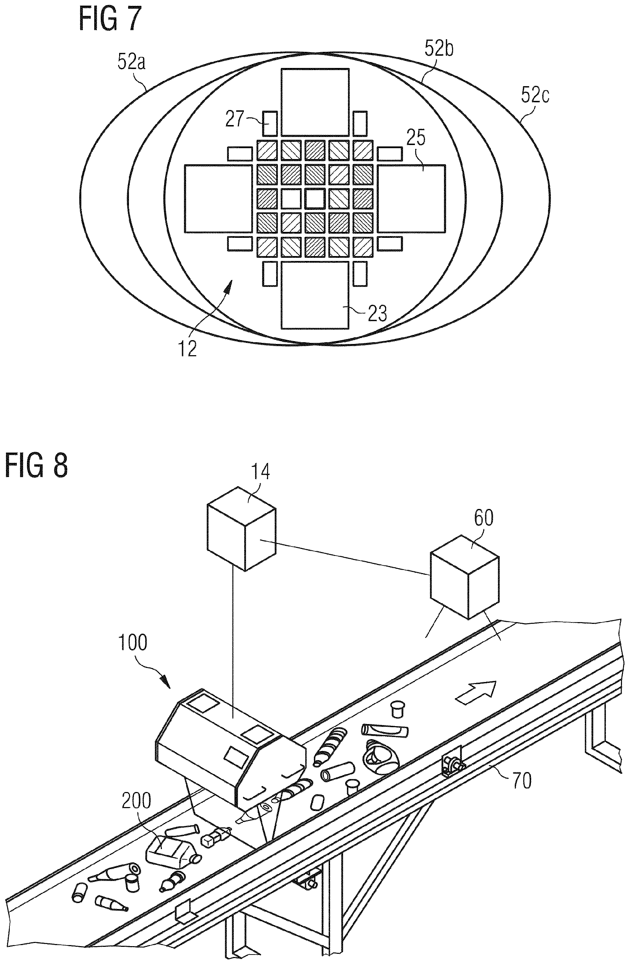

Bei einer anderen Ausführungsform kann die IR-Detektionseinheit 12 ferner eine oder mehrere IR-Detektionsvorrichtungen 23, 25 (siehe Fig. 7), die eine Detektion im NIR-Bereich, beispielsweise zwischen 0,9 μm und 1,7 μm oder zwischen 1,7 μm und 2,55 μm, durchführen, und/oder eine Detektionsvorrichtung 27 für sichtbares Licht aufweisen, wobei der Vergleich mit den Referenzwerten zusätzlich auf den durch diese weiteren Detektionsvorrichtungen detektierten (ggf. integrierten) Intensitäten basiert. Es ist ersichtlich, dass durch Verwendung dieser zusätzlichen Wellenlängen bzw. Wellenlängenbereiche zusätzliche Größen für den Vergleich erhalten werden, so dass der Vergleich feiner und möglicherweise genauer durchgeführt werden kann. Beispielsweise kann so ein Achtbereichsverfahren für die Bestimmung verwendet werden.

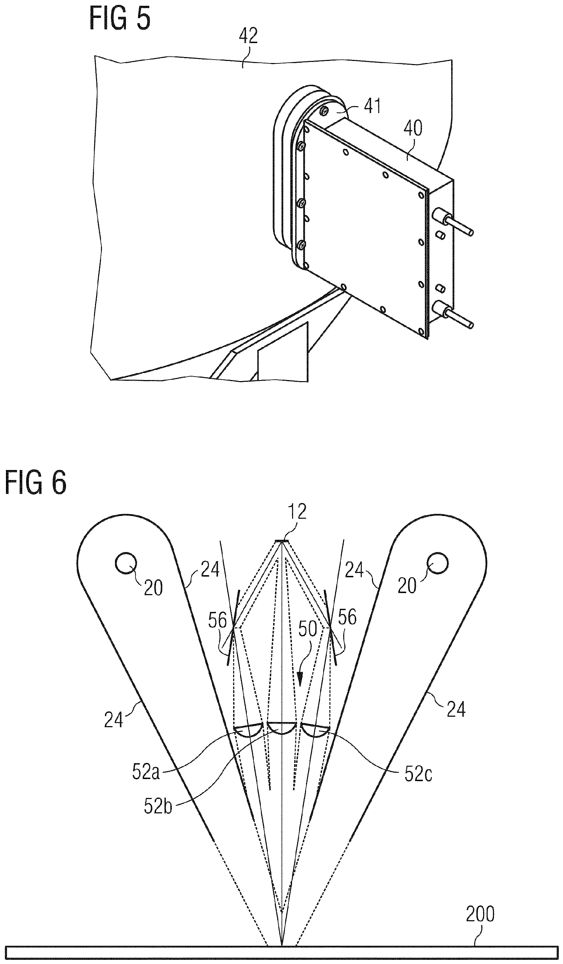

Wie bereits erwähnt, kann die hierin offenbarte Vorrichtung auf besonders vorteilhafte Weise bei der Detektion eines Typs eines Kunststoffmaterials 200 verwendet werden, das bereits in Form eines Granulats vorliegt. Solche Granulate werden üblicherweise in einem Behälter 42, der in Fig. 5 gezeigt ist, transportiert bzw. verarbeitet. Wenn nun, wie in Fig. 5 gezeigt, die Sendeeinheit 10 und die IR-Detektionseinheit 12 in einem gemeinsamen Gehäuse 40 integriert sind, kann eine besonders kompakte Vorrichtung 100 erhalten werden. Dabei ist es besonders vorteilhaft, wenn an dem Gehäuse 40 ein Befestigungsbereich 41, der zur Befestigung an dem Behälter 42 für das Kunststoffmaterial 200 ausgebildet ist, vorgesehen ist und innerhalb des Befestigungsbereichs 41 geeignete Lichtaustritts- bzw. Lichteintrittsbereiche vorgesehen sind. Dabei versteht sich, dass der Behälter 42 an geeigneter Stelle ein zumindest für MIR-Strahlung durchlässiges Fenster aufweisen muss, über das die Detektion durchgeführt werden kann. Beispielsweise kann ein Schauglas 26, wie in Fig. 1 gezeigt, das in Form einer Glasplatte mit $CaF_2$, $BaF_2$ und/oder Si vorliegt, verwendet werden.

Es ist ferner vorgesehen, dass vor der IR-Detektionseinheit 12 eine Optik 50 vorgesehen ist, insbesondere mit einer oder mehreren Linsen 52, die beispielsweise Si, $BaF_2$ oder $CaF_2$ aufweisen oder daraus bestehen. Dies ermöglicht es, die reflektierte Strahlung auf der Detektionseinheit 12 zu fokussieren und so die Intensität der Detektion zu erhöhen. Bei einer in Fig. 6 gezeigten Ausführungsform sind beispielsweise drei Linsen 52a, 52b, 52c vorgesehen, von denen zwei äußere in Bezug auf die innere geneigt sind. Ferner sind zwei Umlenkspiegel 56 vorgesehen, die jeweils den beiden äußeren Linsen zugeordnet sind und zum Lenken des reflektierten IR-Lichts auf einen bestimmten, beispielsweise äußeren, Bereich der IR-Detektionseinheit 12 verwendet werden. Auf diese Weise kann beispielsweise Licht bestimmter Wellenlängen auf bestimmte Detektionsvorrichtungen der IR-Detektionseinheit 12 gelenkt werden.

Fig. 7 zeigt eine beispielhafte Draufsicht auf eine IR-Detektionseinheit 12, bei der die erwähnten drei Linsen 52a, 52b, 52c und zwei Umlenkspiegel vorgesehen sind. Bei der in Fig. 7 gezeigten IR-Detektionseinheit 12 sind neben den zuvor erwähnten IR-Detektionsvorrichtungen 16-19, 21 ferner die zwei IR-Detektionsvorrichtungen 23, 25 für eine Detektion im NIR-Bereich sowie die Detektionsvorrichtung 27 für sichtbares Licht vorgesehen, die im Wesentlichen in einem Randbereich der Matrixanordnung der Detektionsvorrichtungen angeordnet sind. Dabei ist jede der IR-Detektionsvorrichtungen 23, 25 zweifach vorgesehen, beispielsweise einander gegenüberliegend. Durch die Linsen 52a, 52c kann nun gewährleistet werden, dass auch auf diese weiteren, etwas außerhalb vorgesehenen Detektionsvorrichtungen genügend reflektiertes Licht trifft.

Neben der Verwendung der hierin beschriebenen Vorrichtung zur Bestimmung eines Typs bzw. einer Art von zerkleinertem Kunststoffmaterial wie Granulat ist ersichtlich, dass die hierin beschriebene Vorrichtung auch zur Sortierung von größeren Kunststoffobjekten (oder auch zerkleinerten Kunststoffteilen wie sog. Flakes) verwendet werden kann. Wie in Fig. 8 gezeigt, weist die Vorrichtung dazu ferner eine Sortiereinheit 60 auf, die stromabwärts der Sendeeinheit 10 und der IR-Detektionseinheit 12 vorgesehen ist und dazu angepasst ist, von der Steuereinheit 14 basierend auf dem Ergebnis der Bestimmung des Typs des Kunststoffmaterials 200 zum Sortieren desselben, beispielsweise unter Verwendung von geeigneten Ausblasdüsen, auf bekannte Weise angesteuert zu werden. Dabei ist es ersichtlich von Vorteil, wenn beispielsweise mehrere IR-Detektionseinheiten 12 in einer Richtung benachbart zueinander angeordnet sind, so dass eine räumlich aufgelöste Detektion erhalten wird. Dies ist ohne weiteres möglich, indem beispielsweise mehrere der in Fig. 2 gezeigten IR-Detektionseinheiten 12 in einer Richtung im Wesentlichen senkrecht zur Längsrichtung y der Strahler 20 (einer Richtung transversal zu einer Transportrichtung einer Transportvorrichtung 70, beispielsweise eines Förderbands) angeordnet werden. Insbesondere aus Fig. 8 ist ersichtlich, dass eine Verwendung eines Heizstrahlers in Form eines länglichen Rohrs, in Verbindung mit entsprechenden Reflektoren, die ebenfalls in Längsrichtung y des Strahlers verlaufen, auf besonders einfache Weise den gesamten zu detektierenden Bereich beleuchten können.

Mit der oben beschriebenen Vorrichtung bzw. dem zugehörigen Verfahren können auf zuverlässige Weise insbesondere geschwärzte Kunststoffe, seien es bereits zerkleinerte Granulate oder ganze Kunststoffobjekte, die insbesondere schwarz eingefärbt sind, voneinander unterschieden werden.

Es wird explizit betont, dass alle in der Beschreibung und/oder den Ansprüchen offenbarten Merkmale als getrennt und unabhängig voneinander zum Zweck der ursprünglichen Offenbarung ebenso wie zum Zweck des Einschränkens der beanspruchten Erfindung unabhängig von den Merkmalskombinationen in den Ausführungsformen und/oder den Ansprüchen angesehen werden sollen. Es wird explizit festgehalten, dass alle Bereichsangaben oder Angaben von Gruppen von Einheiten jeden möglichen Zwischenwert oder Untergruppe von Einheiten zum Zweck der ursprünglichen Offenbarung ebenso wie zum Zweck des Einschränkens der beanspruchten Erfindung offenbaren, insbesondere auch als Grenze einer Bereichsangabe.

**Patentansprüche**

1. Verfahren zur Detektion eines Typs eines Kunststoffmaterials (200), insbesondere im Rahmen eines Recyclingprozesses, mit

   Aussenden von Anregungslicht, das IR-Licht in einem MIR-Bereich zwischen etwa 2,2 $\mu$m und etwa 5 $\mu$m aufweist, in Richtung des Kunststoffmaterials,
   Detektieren von MIR-Intensitäten IR1, ..., IRn von an dem Kunststoffmaterial reflektiertem IR-Licht in mindestens zwei unterschiedlichen MIR-Wellenlängenbereichen, die in dem MIR-Bereich zwischen etwa 2,2 $\mu$m und etwa 5 $\mu$m liegen,
   Bestimmen des Typs des Kunststoffmaterials durch Vergleichen mindestens der detektierten MIR-Intensitäten mit einer Mehrzahl von Referenzgrößen, die jeweils unterschiedlichen Typen von Kunststoffmaterial zugeordnet sind, und Bestimmen der Referenzgröße mit der größten Übereinstimmung als den Typ des Kunststoffmaterials angebend, ferner mit
   Bestimmen einer Strahlungsleistung des Anregungslichts, beispielsweise basierend auf einer durch einen Temperatursensor (30) erfassten Temperatur,
   Steuern des ausgesandten Anregungslichts basierend auf der bestimmten Strahlungsleistung zum Erhalten eines Soll-Anregungslichts oder
   Modifizieren der detektierten MIR-Intensitäten basierend auf der bestimmten Strahlungsleistung vor dem Vergleichen mit der Mehrzahl von Referenzgrößen.

2. Verfahren nach Anspruch 1, bei dem anhand der detektierten MIR-Intensitäten normierte Intensitäten ir1, ..., irn und optional ein Normierungsfaktor B bestimmt werden, die gegeben sind durch:

$$ir1 = IR1/(IR1 + \ldots + IRn)$$

$$\ldots$$

$$irn = IRn/(IR1 + \ldots + IRn)$$

$$B = (IR1 + \ldots + IRn)/n$$

und
bei dem der Typ des Kunststoffmaterials durch Vergleichen einer Kombination aus den normierten IR-Intensitäten und optional dem Normierungsfaktor mit der Mehrzahl von Referenzgrößen bestimmt wird.

3. Verfahren nach Anspruch 1 oder 2, ferner mit
Aussenden des IR-Lichts unter Verwendung einer breitbandigen IR-Lichtquelle (20) und Detektieren der MIR-Intensitäten durch unterschiedliche, jeweils den mindestens zwei MIR-Wellenlängenbereichen zugeordnete IR-Detektionsvorrichtungen (16-19, 21), beispielsweise IR-Fotodioden.

4. Verfahren nach einem der Ansprüche 1 bis 3, bei dem das Anregungslicht ferner Licht im NIR-Bereich und/oder sichtbaren Bereich aufweist, ferner mit

Detektieren von einer oder mehreren, beispielsweise drei, weiteren Intensitäten I1, ..., In von reflektiertem Licht in dem NIR-Bereich und/oder sichtbaren Bereich und
Bestimmen des Typs des Kunststoffmaterials durch Vergleichen der detektierten MIR-Intensitäten und der detektierten weiteren Intensitäten mit der Mehrzahl von Referenzgrößen.

5. Verfahren nach einem der Ansprüche 1 bis 4, ferner mit

Detektieren eines Spektrums des Anregungslichts,
Steuern des ausgesandten Anregungslichts basierend auf dem detektierten Spektrum zum Erhalten des Soll-Anregungslichts oder
Modifizieren der detektierten MIR-Intensitäten basierend auf dem detektierten Spektrum vor dem Vergleichen mit der Mehrzahl von Referenzgrößen.

6. Verfahren nach einem der Ansprüche 1 bis 5, ferner mit
Sortieren des Kunststoffmaterials (200) basierend auf dem bestimmten Typ.

7. Vorrichtung (100) zur Detektion eines Typs eines Kunststoffmaterials (200), insbesondere im Rahmen eines Recyclingprozesses, mit

einer Sendeeinheit (10) zum Aussenden von Anregungslicht, das IR-Licht in einem MIR-Bereich zwischen etwa 2,2 $\mu$m und etwa 5 $\mu$m aufweist, in Richtung des Kunststoffmaterials, einer IR-Detektionseinheit (12) zum Detektieren von MIR-Intensitäten IR1, ..., IRn von an dem Kunststoffmaterial reflektiertem IR-Licht in mindestens zwei unterschiedlichen MIR-Wellenlängenbereichen, die in dem MIR-Bereich zwischen etwa 2,2 $\mu$m und etwa 5 $\mu$m liegen, einer Steuereinheit (14) zum Bestimmen des Typs des Kunststoffmaterials durch Vergleichen mindestens der detektierten MIR-Intensitäten mit einer Mehrzahl von Referenzgrößen, die jeweils unterschiedlichen Typen von Kunststoffmaterial zugeordnet sind, und Bestimmen der Referenzgröße mit der größten Übereinstimmung als den Typ des Kunststoffmaterials angebend und
mindestens einem Temperatursensor (30),
bei der die Steuereinheit (14) angepasst ist zum Bestimmen einer Strahlungsleistung des Anregungslichts basierend auf einer durch den mindestens einen Temperatursensor (30) erfassten Temperatur und Steuern der IR-Lichtquelle (20) basierend auf der bestimmten Strahlungsleistung zum Erhalten eines Soll-Anregungslichts oder Modifizieren der detektierten MIR-Intensitäten basierend auf der bestimmten Strahlungsleistung vor dem Vergleichen mit der Mehrzahl von Referenzgrößen.

8. Vorrichtung nach Anspruch 7, bei der die Sendeeinheit (10) eine breitbandige IR-Lichtquelle (20) aufweist und die IR-Detektionseinheit (12) unterschiedliche, jeweils den mindestens zwei MIR-Wellenlängenbereichen zugeordnete IR-Detektionsvorrichtungen (16, 18), beispielsweise IR-Fotodioden, aufweist.

9. Vorrichtung nach Anspruch 8, bei der die breitbandige IR-Lichtquelle (20) ein Infrarot-Heizstrahler ist, der sich in einer Längsrichtung (y) erstreckt, insbesondere ein Quarzstrahler, der einen in ein mit Inertgas gefülltes Quarzglasrohr integrierten Heizwiderstand (22) aufweist, oder ein flächiger Infrarotstrahler.

10. Vorrichtung nach einem der Ansprüche 7 bis 9, bei der die Sendeeinheit (10) einen Reflektor (24) aufweist, der von der IR-Lichtquelle (20) emittiertes Licht in Richtung eines Detektionsbereichs (26) der Vorrichtung (100) lenkt, beispielsweise einen Parabolspiegel, der die IR-Lichtquelle (20) teilweise umgibt, und/oder mehrere Bleche, die in Richtung des Detektionsbereichs aufeinander zulaufen, so dass ein schlitzförmiger Austrittsbereich (28) der Sendeeinheit (10) erhalten wird.

**11.** Vorrichtung nach einem der Ansprüche 7 bis 10, ferner mit mindestens einem Detektor (32), der dazu angepasst ist, ein Spektrum des Anregungslichts zu detektieren,
bei der die Steuereinheit (14) angepasst ist zum Steuern des ausgesandten Anregungslichts basierend auf dem detektierten Spektrum zum Erhalten eines Soll-Anregungslichts oder Modifizieren der detektierten MIR-Intensitäten basierend auf dem detektierten Spektrum vor dem Vergleichen mit der Mehrzahl von Referenzgrößen.

**12.** Vorrichtung nach einem der Ansprüche 7 bis 11, bei der die Detektionseinheit (12) eine oder mehrere IR-Detektionsvorrichtungen (23, 25), die eine Detektion im NIR-Bereich, beispielsweise zwischen 0,9 $\mu$m und 1,7 $\mu$m oder zwischen 1,7 $\mu$m und 2,55 $\mu$m, durchführen, und/oder eine Detektionsvorrichtung (27) für sichtbares Licht aufweist, wobei der Vergleich mit den Referenzwerten zusätzlich auf den durch diese weiteren Detektionsvorrichtungen detektierten Intensitäten basiert.

**13.** Vorrichtung nach einem der Ansprüche 7 bis 12, bei der die Sendeeinheit (10) und die IR-Detektionseinheit (12) in einem gemeinsamen Gehäuse (40) integriert sind, insbesondere mit einem an dem Gehäuse (40) ausgebildeten Befestigungsbereich (41), der zur Befestigung an einem Behälter (42) für das Kunststoffmaterial (200) ausgebildet ist, und einem innerhalb des Befestigungsbereichs (41) vorgesehenen Lichtaustritts- bzw. Lichteintrittsbereich.

**14.** Vorrichtung nach einem der Ansprüche 7 bis 13, ferner mit einer Sortiereinheit (60), die dazu angepasst ist, von der Steuereinheit (14) basierend auf dem Ergebnis der Bestimmung des Typs des Kunststoffmaterials (200) zum Sortieren desselben angesteuert zu werden.

**15.** Vorrichtung nach einem der Ansprüche 7 bis 14, bei der mehrere IR-Detektionseinheiten (12) in einer Richtung benachbart zueinander angeordnet sind, so dass eine räumlich aufgelöste Detektion erhalten wird.

FIG 1

FIG 2

FIG 3

21    34

12

18

16

19

17

FIG 4a

P16612-045CF

P16612-043CF   (Typ. Ta=25°C)

P16612-039CF

4.0
3.5
3.0
2.5
2.0
1.5
1.0
0.5
0

Sensitivität [mA/W]

2.5   3.0   3.5   4.0   4.5   5.0

P16612-
033CF          Wellenlänge [μm]

FIG 4b

(Typ. Ta=25°C)

$10^{10}$

P16612-011CA/CN
P16849-013CN

$10^{9}$

D* [cm·$Hz^{1/2}$/W]

$10^{8}$

P16112-011MA

$10^{7}$

2   3   4   5   6

Wellenlänge [μm]

## FIG 5

## FIG 6

FIG 7

FIG 8

## EUROPÄISCHER RECHERCHENBERICHT

**Nummer der Anmeldung**

EP 24 17 2150

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2023/007824 A1 (DAIO SEISHI KK [JP]) 2. Februar 2023 (2023-02-02) | 1,2,5,6 | INV. G01N21/3563 |
| Y | * Absätze [0055] - [0075]; Abbildungen 1-2,6-10 * | 3,4, 7-12,14 | B29B17/02 B07C5/342 G01N21/85 |
| Y | EP 3 477 282 B1 (PANASONIC IP MAN CO LTD [JP]) 9. Februar 2022 (2022-02-09) * Absätze [0030] - [0053], [0066] - [0069]; Abbildungen * | 1-5,7-9, 11-13 | |
| Y | HOGSTEDT LASSE ET AL: "Identification of black plastics using an upconversion based mid-infrared imaging spectrograph", 2017 CONFERENCE ON LASERS AND ELECTRO-OPTICS EUROPE & EUROPEAN QUANTUM ELECTRONICS CONFERENCE (CLEO/EUROPE-EQEC), IEEE, 25. Juni 2017 (2017-06-25), Seite 1, XP033239769, DOI: 10.1109/CLEOE-EQEC.2017.8086844 [gefunden am 2017-10-26] * das ganze Dokument * | 1-12,14, 15 | |
| Y | WOLFGANG BECKER ET AL: "Detection of Black Plastics in the Middle Infrared Spectrum (MIR) Using Photon Up-Conversion Technique for Polymer Recycling Purposes", POLYMERS, Bd. 9, Nr. 435, 8. September 2017 (2017-09-08), XP055537028, DOI: 10.3390/polym9090435 * das ganze Dokument * | 1-9,11, 12,14 | |

**RECHERCHIERTE SACHGEBIETE (IPC)**

G01N
B07C
B29B

-/--

Der vorliegende Recherchenbericht wurde für alle Patentansprüche erstellt

| Recherchenort | Abschlußdatum der Recherche | Prüfer |
|---|---|---|
| München | 20. September 2024 | Riblet, Philippe |

# EUROPÄISCHER RECHERCHENBERICHT

Europäisches Patentamt
European Patent Office
Office européen des brevets

Nummer der Anmeldung

EP 24 17 2150

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | US 5 510 619 A (ZACHMANN GUENTER [DE] ET AL) 23. April 1996 (1996-04-23) * Zusammenfassung; Abbildungen * * Spalte 7, Zeilen 20-55 * * Spalte 7, Zeile 64 - Spalte 8, Zeile 6 * ----- | 1-9, 11-14 | |
| Y | US 10 375 327 B2 (REBELLION PHOTONICS INC [US]) 6. August 2019 (2019-08-06) * Spalte 29, Zeilen 25-46 * ----- | 1-15 | |
| Y | CN 203 465 005 U (BEIJING AEROSPACE INST FOR METROLOGY & MEASUREMENT TECHNOLOGY ET AL.) 5. März 2014 (2014-03-05) * Absatz [0032] * ----- | 1-15 | |
| Y | US 2013/110311 A1 (VER STEEG BENJAMIN [US] ET AL) 2. Mai 2013 (2013-05-02) * Abbildung 33 * ----- | 1-15 | |
| Y | WO 2022/170262 A1 (SORTERA ALLOYS INC [US]) 11. August 2022 (2022-08-11) * Abbildung 4 * ----- | 1,3,4,8, 9,12 | RECHERCHIERTE SACHGEBIETE (IPC) |
| A | HERMES M ET AL: "Mid-IR hyperspectral imaging for label-free histopathology and cytology", JOURNAL OF OPTICS, INSTITUTE OF PHYSICS PUBLISHING, BRISTOL GB, Bd. 20, Nr. 2, 24. Januar 2018 (2018-01-24), Seite 23002, XP020324038, ISSN: 2040-8986, DOI: 10.1088/2040-8986/AAA36B [gefunden am 2018-01-24] * das ganze Dokument * ----- | 1-15 | |
| A | US 2005/270528 A1 (GESHWIND FRANK [US] ET AL) 8. Dezember 2005 (2005-12-08) * Zusammenfassung; Abbildungen * ----- | 1-15 | |

Der vorliegende Recherchenbericht wurde für alle Patentansprüche erstellt

| Recherchenort | Abschlußdatum der Recherche | Prüfer |
|---|---|---|
| München | 20. September 2024 | Riblet, Philippe |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
............................................................................
& : Mitglied der gleichen Patentfamilie, übereinstimmendes Dokument

EPO FORM 1503 03.82 (P04C03)

Seite 2 von 2

**ANHANG ZUM EUROPÄISCHEN RECHERCHENBERICHT
ÜBER DIE EUROPÄISCHE PATENTANMELDUNG NR.**

EP 24 17 2150

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

20-09-2024

| Im Recherchenbericht angeführtes Patentdokument | Datum der Veröffentlichung | Mitglied(er) der Patentfamilie | Datum der Veröffentlichung |
|---|---|---|---|
| WO 2023007824 A1 | 02-02-2023 | JP 2023017263 A<br>WO 2023007824 A1 | 07-02-2023<br>02-02-2023 |
| EP 3477282 B1 | 09-02-2022 | CN 109719055 A<br>EP 3477282 A1 | 07-05-2019<br>01-05-2019 |
| US 5510619 A | 23-04-1996 | DE 4340914 A1<br>GB 2284477 A<br>US 5510619 A | 08-06-1995<br>07-06-1995<br>23-04-1996 |
| US 10375327 B2 | 06-08-2019 | CA 3041105 A1<br>CN 110073185 A<br>CN 114609075 A<br>EP 3529573 A1<br>US 2018191967 A1<br>US 2020128196 A1<br>US 2021250525 A1<br>WO 2018075964 A1 | 26-04-2018<br>30-07-2019<br>10-06-2022<br>28-08-2019<br>05-07-2018<br>23-04-2020<br>12-08-2021<br>26-04-2018 |
| CN 203465005 U | 05-03-2014 | KEINE | |
| US 2013110311 A1 | 02-05-2013 | CN 104039577 A<br>EP 2750912 A1<br>EP 3858651 A1<br>JP 2014529467 A<br>JP 2016202975 A<br>JP 2019049570 A<br>JP 2021101196 A<br>JP 2023100854 A<br>US 2013110311 A1<br>US 2017050518 A1<br>US 2019275886 A1<br>WO 2013033099 A1 | 10-09-2014<br>09-07-2014<br>04-08-2021<br>13-11-2014<br>08-12-2016<br>28-03-2019<br>08-07-2021<br>19-07-2023<br>02-05-2013<br>23-02-2017<br>12-09-2019<br>07-03-2013 |
| WO 2022170262 A1 | 11-08-2022 | EP 4288219 A1<br>EP 4288220 A1<br>JP 2024508684 A<br>JP 2024510084 A<br>KR 20230159398 A<br>KR 20230159399 A<br>TW 202301192 A<br>WO 2022170262 A1<br>WO 2022170273 A1 | 13-12-2023<br>13-12-2023<br>28-02-2024<br>06-03-2024<br>21-11-2023<br>21-11-2023<br>01-01-2023<br>11-08-2022<br>11-08-2022 |
| US 2005270528 A1 | 08-12-2005 | KEINE | |

Für nähere Einzelheiten zu diesem Anhang : siehe Amtsblatt des Europäischen Patentamts, Nr.12/82

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102020113252 A1
- WO 2023017639 A1
- WO 2022170262 A1
- DE 69307344 T2